# EUROPEAN PATENT SPECIFICATION

(11) **EP 3 838 228 B1**
(45) Date of publication and mention of the grant of the patent: **31.01.2024**
(21) Application number: 19216467.1
(22) Date of filing: 16.12.2019
(51) Int. Cl.: A61F 2/38, A61B 17/72, A61F 2/30

(54) **JOINT PROSTHESIS AND ASSOCIATED METHOD OF ASSEMBLY**
GELENKPROTHESE UND ZUGEHÖRIGES MONTAGEVERFAHREN
PROTHÈSE D'ARTICULATION ET MÉTHODE D'ASSEMBLAGE ASSOCIÉE

(43) Date of publication of application: 23.06.2021
(73) Proprietor: Waldemar Link GmbH & Co. KG, 22339 Hamburg (DE)
(72) Inventor: DALLA PRIA, Paolo, 33100 Udine (IT); SCHULTZ, Thomas, 21365 Adendorf (DE)
(74) Representative: AWA Denmark A/S

(56) References cited:
- EP-A1- 3 054 896
- US-A1- 2016 220 378
- US-B1- 10 278 824
- US-B2- 9 044 325

## Description

### FIELD OF THE INVENTION

The present invention is defined in claims 1 and 14 and relates to a joint prosthesis and a method for assembling, in particular pre-assembling, such a prosthesis.

### BACKGROUND OF THE INVENTION

One type of joint that enables the skeleton to move is the synovial joint that comprises articulating surfaces that are separated from each other in a synovial cavity surrounded by a fibrous joint capsule. Some of these synovial joints are particularly stabilized by soft tissue structures such as the elbow joint, the knee joint, the ankle joint (talocrural joint), and the wrist joint. With the exception of the knee joint, a replacement of these joints with a joint replacement is a less common surgical procedure than the replacement of the oldest type of artificial joint replacement, i. e. the hip prosthesis. One reason for this may well be that these joints have a rather complex configuration, which makes the design of an artificial joint replacement as well as the surgical procedure to implant such a replacement more challenging.

For example, the elbow joint is essentially a hinge joint, it involves three long bones, namely the humerus, the radius and the ulna. Every one of these bones interacts with the other two bones via a synovial joint. The humeroulnar joint and the humeroradial joint together form the elbow joint.

In addition, the proximal radioulnar joint as the third joint enables pronation and supination of the hand. These are rotational movements that occur when the distal end of the radius moves over the distal end of the ulna, i. e. when the forearm rotates about its longitudinal axis. Although the proximal radioulnar joint is not considered as being part of the elbow joint in an anatomical sense, it is close enough to the elbow joint to be potentially affected during implantation of a joint replacement.

Such a replacement of the elbow joint or any of the other joints listed above is, for example, considered in severe cases of arthritis. The pain felt due to arthritis in these joints results in a significant impact on the life of a patient, which may be explained by the frequent movements of the arms and the necessity of these movements during everyday life. Here, the replacement of the affected joint generally offers the prospect of a pain-free life after surgery.

There are several techniques available to replace a native joint, such as a surface replacement or a total joint replacement. In the former case, the damaged cartilage surface is replaced by an artificial surface. With this technique, the joint surfaces remain separate, which requires to keep the ligaments surrounding and supporting the joint as intact as possible in order to prevent the partly replaced joint from becoming unstable after surgery. Nonetheless, in practice, it turned out that keeping the ligaments intact to such a degree is rather hard so that a common problem with this type of replacement is a luxation of the joint after surgery.

These problems in terms of stability also occur with total joint replacements that are not mechanically linked. This type of prosthesis replaces the ends of the bones facing each other, where the native joint is normally located, with implant components.

For this reason, in particular the elbow joint is often replaced with a total elbow replacement that is mechanically connected. Such a total elbow replacement has the form of a connected hinge joint and is attached on one side to the humerus and on the other side to the ulna. As a result, the humeroradial joint is resected but not replaced since the hinge joint of a total elbow replacement for most joint replacements only involves the humeroulnar joint. Although this design prevents luxation from occurring, this type of replacement has been shown to be subject to a significant amount of wear. It is believed that this is a result of the difference between the kinematics of the total elbow replacement and the native elbow joint and an insufficient adaptation of a patient's body to this difference.

Similar to the elbow joint, the other above-noted joints have a dominant rotational degree of freedom with a large range of motion but also allow for a rotation to a less degree in another direction such as a varus-valgus movement. For the reasons described above in relation to the elbow replacement, it is believed that replacing these joints with mechanically connected total replacements likewise increases stress and wear on their components.

In answer to this, less strict hinge joints have been developed as total elbow replacements that offer more leeway in their movability.

The prior art document US 10,195,040 B2 discloses an elbow prosthesis including a humeral component having a yoke and an ulnar component allowing a flexural movement of the forearm, i.e. a pivotal movement, while at the same time gives leeway so as to allow a varus-valgus movement. This varus-valgus range of motion, however, renders the disclosed prosthesis less stable and prone to wear. More specifically, the joint surfaces are loosely connected to each other, i. e. they form a wobbly joint, in order to increase the joint's range of motion to include a varus-valgus movement.

US 2016/0338839 A1 discloses a prosthetic elbow joint providing for pivotal movement while at the same time providing varus-valgus movement, however, the disclosed device has the downside of free play between the ulnar and humeral component. Although this play allows for said varus-valgus movement, this play results in a wobbly or sloppy hinge with partial contact between the joint surfaces. Such a joint has a designed instability and shows an increased amount of wear.

Thus, more excessive wear and problems of loosening than with hip joint replacements are still being observed.

US 9 044 325 B2 and US 10 278 824 B1, both of which disclose the technical features of the preamble of claim 1, state an elbow prosthesis which includes an ulnar component that has a bearing end and a stem, wherein the stem is attached to the bearing end and extends in a distal direction from it. The elbow prosthesis also has a humeral component that includes a holder end and a stem with the stem extending in a proximal direction from the holder end. The prosthesis further includes at least one bearing member that is connected to the holder end with the bearing end being attached within the holder end to allow for rotation and articulation against the at least one bearing member.

US 2016/220378 A1 is related to an elbow arthroplasty prosthesis that includes an ulnar component, a humeral component, one or more articulation liners, and a retention trap, wherein the ulnar component includes a spherical bearing head that can be inserted into the humeral component in a number of orientation and the articulation liners and the retention trap are operatively engaged to the humeral component to retain the ulnar component within a humeral socket.

### SUMMARY OF THE INVENTION

It is, in view of the prior art, an object of the present invention to provide a system or mechanism for the replacement of particularly the above-noted synovial joints, which allows for a rotation in the dominant rotational direction while at the same time provides sufficient stability and reduces wear and stress. It is an additional object of the present invention to provide a system or mechanism for the replacement of such a joint that allows for an easy assembly.

This problem is solved by a joint prosthesis according to claim 1 and a method of assembly of the claimed joint prosthesis in accordance with claim 14.

The dependent claims relate to advantageous embodiments of the invention.

A joint prosthesis according to the present invention comprises a first component and a second component being connected via a ball-and-socket-joint, wherein the first component comprises two outward (i. e. convex) spherical sections, at least one of the spherical sections having a projection. The ball-and-socket-joint comprises two gliding blocks each having an inward (i. e. concave) spherical section and at least one of the gliding blocks comprising a hole, the gliding blocks being attached to the second component, and the outward spherical sections are in gliding engagement with the inward spherical sections of the gliding blocks so that the first component is movably attached to the second component and the projection is inserted into one of the holes of the gliding blocks, so as to limit the mobility of the ball-and-socket-joint. An inner diameter of the hole is larger than an outer diameter of the projection.

This joint prosthesis allows for flexural, i.e. pivotal movement, while at the same time provides a defined varus-valgus movement. The spherical sections of the first component and the gliding blocks allow for an increased contact surface of the moving parts, because with the spherical sections larger surfaces of the joint remain in contact during the varus-valgus movement. Thereby, stresses and wear are reduced and stability is enhanced.

In the present context, the term "outward spherical section" means a spherical section bulging outwardly from the component to which the outward spherical section is attached. In other words, such an outward spherical section is convex.

The term "inward spherical section" relates to a spherical section that in contrast to the outward bulging spherical section is formed as a concavity or depression in the corresponding component. In other words, such an inward spherical section is concave.

The hole of a gliding block may be a fully or partially penetrating hole.

The term gliding engagement relates to an engagement of a joint surface of the first component with a corresponding joint surface of the second component's gliding blocks, which allows for the first component to be moved relative to the second component while at the same time being in contact with the gliding blocks. Further, the joint surfaces are engaged to a degree that they do not separate. In other words, they mechanically connect the first component and the second component. This mechanical connection substantially prevents a translation but allows for a rotation between these components.

Limitation of the mobility of the ball-and-socket-joint by the projection in the present case relates to the varus-valgus movement and the rotation of the first component about a second pivotal axis in these directions. The projections do not limit the dominant or major pivotal or flexural movement of the joint about a first pivotal axis.

In conventional joint prostheses allowing for a varus-valgus movement, the contact surface of the moving parts is greatly reduced as the contact portions of the first component being at the maximum varus position are in front of the pivotal axis of the joint on one side of the joint and behind the rotational axis on an opposing side of the joint. When considering the maximum valgus position this configuration switches and the contact portions of the first component are behind the pivotal axis of the joint on the one side of the joint and in front of the rotational axis on said opposing side of the joint.

The present invention allows to provide a gliding contact of the first component around the rotational axis (first pivotal axis of the joint) because the spherical sections remain in gliding engagement and the contact of the moving parts of the joint prosthesis do basically not change during a varus-valgus movement.

The at least one projection inserted into the gliding block allows for a well-defined limitation of the varus-valgus movement and, thus, provides a limit to the rotation of the varus-valgus movement of the first component independent of the pivotal position of the joint about the first pivotal axis. By providing such a movement, the wear of the joint can be reduced whereas the engagement and the limitation of the varus-valgus movement prevents the unstable characteristics of a wobbly joint.

It is preferred that the projection has a conical shape with the end of the conical projection away from the spherical section being smaller in circumference than the end of the conical projection at the spherical section. In other words, the projection tapers in its direction of extension from the spherical section.

By forming the projection in a conical shape, the contact surface within the gliding block is enhanced so that load peaks are reduced as the varus-valgus movement is approaching its maximum deflection.

It is further preferred that the second one of the two outward spherical sections has a second projection and the second of the two gliding blocks also comprises a hole into which hole the second projection is inserted. Preferably, the second projection has a conical shape with the end of the conical projection away from the spherical sections being smaller in circumference than the end of the conical projections at the spherical section, i. e. tapering when extending outwards. This also allows to reduce load peaks as the prosthesis approaches its maximum varus-valgus movement.

The second component may comprise two mounting recesses into which the two gliding blocks are inserted and are attached to, respectively. By providing two mounting recesses for inserting the gliding blocks, the assembly is simplified and requires less space while at the same time the gliding blocks are arranged so as to provide a well-defined support for the first component.

Preferably, the gliding blocks are secured with a screw in the mounting recesses. Alternatively, the gliding blocks may be secured to the second component using a form-fit and, in particular, a snap-fit. These configurations allow securing the gliding blocks in an easy fashion while at the same time allowing for both easy assembly and disassembly of the prosthesis.

The mounting recesses may comprise a projection to prevent a rotational movement of the gliding blocks. Preventing a rotational movement of the gliding blocks within the mounting recesses allows aligning the gliding blocks upon assembly to further facilitate assembly of the prosthesis. At the same time this allows for a defined alignment of the mounting recess and the gliding block.

It is further preferred that the mounting recesses comprise an insertion stop to limit insertion of the gliding blocks into the mounting recesses, the means preferably being a step or a pin. By providing such an insertion stop to limit insertion of the gliding blocks into the mounting recesses, the assembly is further simplified. Additionally, the degree of engagement of the outward spherical sections with the inward spherical sections can be precisely set in advance by adjusting the depth of insertion of the gliding blocks. Thereby the force applied to the first component by the gliding blocks can be adjusted.

Preferably, the first component comprises a bone member and a joint member so that the first component can be adjusted to the size of the bone of the patient, thereby providing for better adjustment and anchorage of the prosthesis to the adjacent bone.

Preferably, the two outward spherical sections of the first component are arranged so that they form a spherical convex joint surface. Likewise, the two inward spherical sections of the gliding blocks of the second component are preferably arranged so that they form a spherical concave joint surface. The spherical convex joint surface and the spherical concave joint surface correspond to each other in order to form the ball-and-socket-joint. This enhances the stability and durability of the prosthesis while at the same time reduces wear.

Preferably, the second component comprises one or two cut-out portions through which the at least one projection of the first component can be inserted at the time of assembly. By comprising cut out portions, the first component can be inserted into the second component without the necessity of a tilting movement, thereby further facilitating assembly of the joint prosthesis.

The diameter of curvature of the outward spherical sections and the diameter of the curvature of the inward spherical sections may preferably substantially be equal. This allows to enhance the contact between the first component and the gliding blocks.

Preferably, the joint member of the first component of the joint prosthesis may be formed as one member with the outward spherical sections and the at least one projection, so as to form one unitary part. This facilitates assembly of and provides strength to the joint prosthesis.

The joint prosthesis is preferably an elbow joint prosthesis, in particular with the first component being an ulnar component and the second component being a humeral component.

According to a different aspect of the present invention, a method of assembly for a joint prosthesis is provided comprising introduction of the first component into the second component and subsequently movably fixing the first component by inserting the gliding blocks. After insertion of the gliding blocks, the first component and second component basically prevent translation but allow rotation in relation to each other.

Preferably, the method of assembly further comprises securing the gliding blocks in the second component via one or more screws.

Further features and advantages of the invention are explained in the following description of preferred embodiments and are illustrated in the drawings.

### BRIEF DESCRIPTION OF THE DRAWINGS

The following figures illustrate preferred embodiments of the present invention. These embodiments are not to be construed as limiting but merely for enhancing the understanding of the invention in context with the following description.
FIG. 1A shows a top view of an elbow joint prosthesis as an exemplary embodiment of a joint prothesis according to the present disclosure;
FIG. 2 is a perspective drawing of a humeral component acting as a second component of the joint prosthesis according to the present disclosure;
FIG. 3A is a perspective drawing of a ulnar component acting as a first component of the joint prosthesis according to the present disclosure;
FIG. 3B depicts the joint member of the ulnar component;
FIGs. 4A and 4B are perspective views showing the gliding blocks;
FIG. 5 is a perspective drawing showing the gliding blocks in engagement with the joint member of the ulnar component;
FIG. 6 shows the configuration of FIG. 5 with the humeral component holding the gliding blocks;
FIG. 7A schematically shows how the range of motion of the ulnar component is limited by projections;
FIG. 7B shows the varus-valgus movement of the ulnar component; and
FIGs. 8A, 8B and 8C show the assembly steps necessary for the joint prosthesis.

### DESCRIPTION OF PREFERRED EMBODIMENTS

In the appended drawings, the same reference numerals will be used for similar or the same elements. A repetitive description of these elements will be omitted. Therefore, description of an element may refer not only to a particular figure but several or all figures.

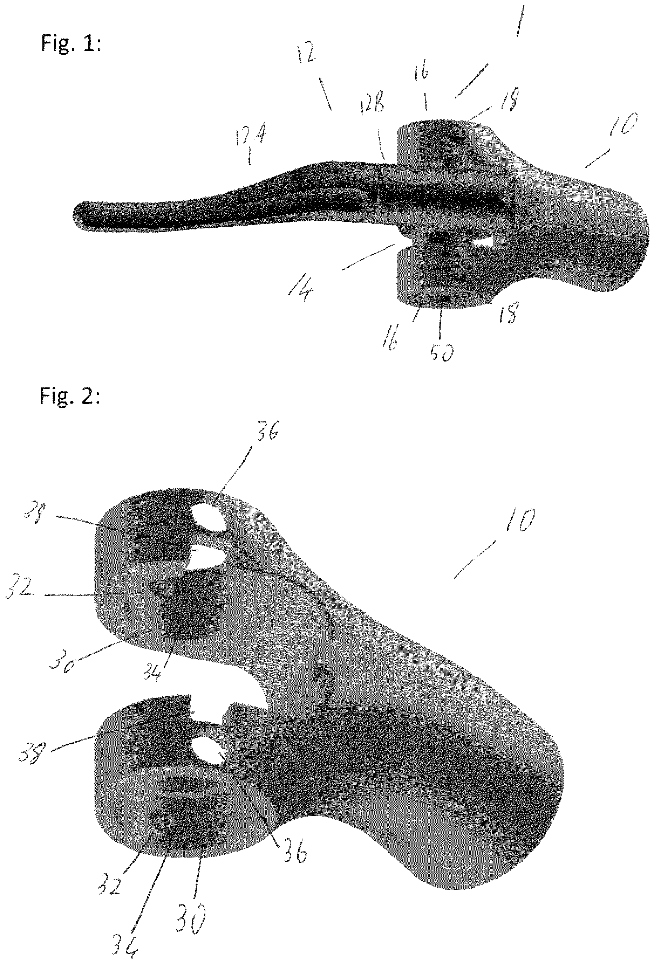

Fig. 1 shows a joint prosthesis 1 comprising a first component 12, a second component 10 and a ball-and-socket-joint 14. The exemplary joint prosthesis illustrated in the figures and described in the following is configured as an elbow joint prosthesis. The first component 12 and the second component 10 of the joint prosthesis are configured for anchorage to the adjacent bones.

More specifically, the first component 12 of the exemplary embodiment is configured as an ulnar component that preferably comprises a bone member 12A and a joint member 12B. The bone member 12A is to be anchored to the ulna of a patient upon implantation of the prosthesis. Accordingly, the second component 10 is configured as a humeral component and comprises a joint member and a bone member (not shown), wherein the bone member is configured for being anchored to the humerus. The skilled person will appreciate that the first component 12 and second component 10 may also be configured vice versa, i. e. the first component 12 may be configured as a humeral component and the second component 10 may be configured as an ulnar component.

Nonetheless, the configuration as an elbow joint prosthesis is an exemplary embodiment. The configuration of the joint prosthesis of this disclosure is more generally directed joints that have a combination of a large range of motion in one rotational direction and a much smaller range of motion in another rotational direction. For example, the joint prosthesis 1 may alternatively be configured as a knee joint prosthesis, an ankle joint prosthesis, or a wrist joint prosthesis.

Like the elbow joint prosthesis, the first component 12 and the second component 10 in these alternative embodiments are also adapted to be anchored to the bone tissue adjacent to the joint prosthesis 1. The bone members of any of these joint prosthesis configurations may be anchored indirectly via bone cement or directly via bone ingrowth.

As illustrated in Fig. 1, the joint member 12B is connected to the second component 10 via the gliding blocks 16. The gliding blocks 16 are preferably secured/fixed to the second component 10 by means of screws 18.

In the following paragraphs the second component 10, the first component 12 and the gliding blocks 16 will be individually described with reference to FIGs. 2 to 4B.

The second component 10 is y- or yoke-shaped and comprises two mounting recesses 30 facing each other. More specifically, one mounting recess 30 is formed through each of the two branches of the yoke-shaped second component 10. Each mounting recess 30 may comprise an insertion stop such as a step 34 or any of the other features described further below for limiting insertion of a gliding block 16.

Transverse to the mounting recess 30, the second component 10 may include a through hole 36 in which a screw 18 can be inserted upon assembly for securing the gliding block 16 within the mounting recess 30.

Further, an indexing structure for preventing a rotation of the guiding blocks 16 arranged in the mounting recesses 30. For example, a projection 32 for engaging a corresponding recess (not shown) of the gliding block 16 may be provided for hindering the rotation of the gliding block 16.

In the illustrated embodiment, each mounting recess 30 comprises a step 34. The step acts as an abutment surface for the gliding blocks 16, once they are inserted into the mounting recess 30 of each branch from the outside towards the inside of the joint prosthesis 1. In other words, the gliding blocks 16 can be pushed into the mounting recess 30 until the gliding blocks 16 abut against said step 34.

While the illustrated embodiment shows a step 34 in the mounting recesses 30 of the second component 10, alternative means to limit insertion of the gliding blocks 16 are conceivable. For example, instead of a step 34 a protrusion or a pin limiting insertion of the gliding block 16 may be used.

As yet another alternative, the gliding blocks may comprise a protrusion or the like and the second component 10 may comprise a kerf, groove or cut-out portion so that insertion of the gliding blocks 16 is limited when the projection of the gliding block 16 reaches the end of the groove, cut-out portion or kerf.

As mentioned above, each mounting recess 30 may further comprise a projection 32 for preventing a rotation of the gliding blocks 16. Accordingly, the gliding blocks 16 comprise a recess 58 or groove, which engages the projection 32 upon insertion of the gliding block 16 into the mounting recess 30 of the second component 10. However, the present invention is not limited to said configuration and alternatively the gliding block 16 may comprise a projection while the second component 10 comprises a corresponding recess.

To facilitate the insertion of the first component 12 and specifically the joint member 12B thereof into the second component 10, cut-out portions 38 are preferably formed in each of the mounting recesses 30. This facilitates insertion of the joint member 12B of the first component 12 and in particular projections 44 of this component described in more detail below into the second component 10 since the cut-out portions 38 at least reduce the necessity of having to rotate or twist the first component 12 during assembly.

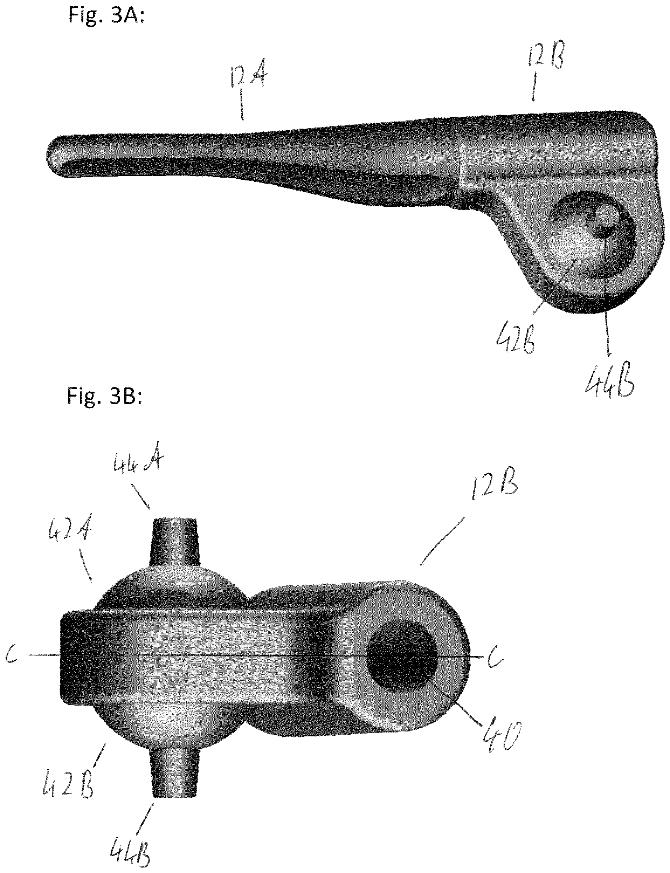

The first component 12 comprises a bone member 12A and a joint member 12B. The joint member 12B comprises outward spherical sections 42. FIGs. 3A and 3B show spherical sections 42A and 42B both of which spherical sections 42 also comprise a projection 44A and 44B, respectively. The projections 44 define a first pivotal axis of the first component 12 for the dominant degree of freedom in terms of range of motion.

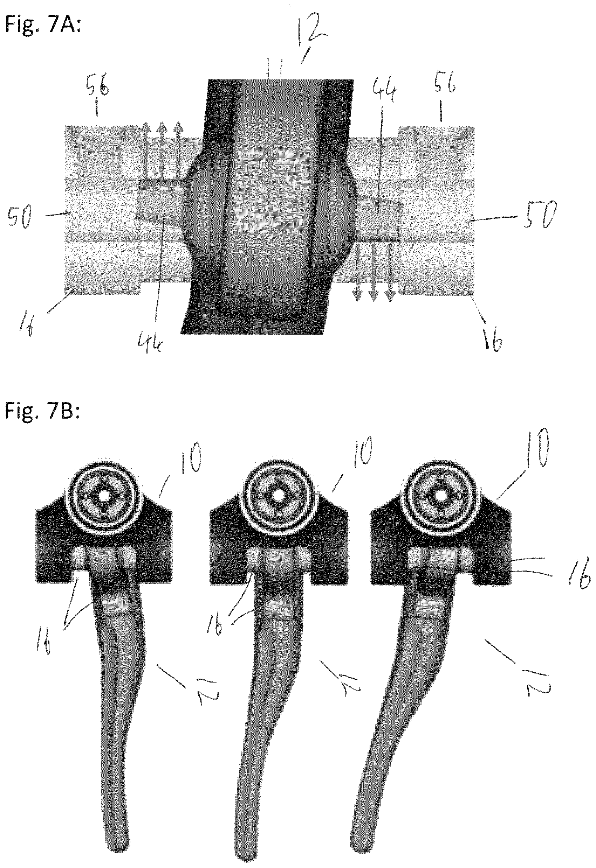

In the illustrated embodiment, the two projections 44A and 44B have a conical shape with the circumference of the projections 44 being reduced the further the projection is away from the spherical sections 42. The present invention is, however, not limited to conically shaped projections and any shape of projection is conceivable, as long as the projection can limit the range of motion of the joint, as will be further explained with reference to FIG. 7A.

The joint member 12B of the first component 12 preferably comprises an interface part for engaging the bone member 12A. In the illustrated embodiment, this interface is configured as hole 40 for inserting a corresponding interface part of the bone member 12A, the interface part of the bone member 12A being preferably formed as a protrusion. The interface between the bond member 12A and the joint member 12B is preferable configured as a conical connection. As already described above, the configuration of the bone members of the joint prothesis 1 depends on the bone tissue the bone member is to be anchored to. For example, for an adjacent long bone, a bone member is preferably anchored using an implant stem such as the implant stem of the bone member 12A illustrated in the figures. For smaller bones other structures may be used such as fins, pins, or screws.

As depicted in FIG. 3B, the outward (i. e. convex) spherical sections 42A and 42B are extending in opposite directions and are symmetrical in relation to a plane C so as to form a convex spherical joint surface. This joint surface is configured to interact with a concave spherical joint surface formed by the inward spherical sections 52 of the gliding block via a surface contact. They are preferably formed as an integral part of the joint member 12B. The spherical joint surface of the outward spherical sections 42 enhances the stability and reduces wear of the joint prosthesis 1 as it provides for a varus-valgus movement about a second pivotal axis of the joint while substantially keeping a surface contact area. In other words, this configuration allows for a rotation about the first and second pivotal axes without a substantial change of the size of the surface contact area. Rotation about the first pivotal axis of the first component 12 allows for a range of motion of the joint in flexion-extension (e. g. for an elbow joint in a range of 0° to 160°). Rotation about the second pivotal axis of the first component 12 allows for a smaller range of motion in varus-valgus (e. g. approximately ±5°).

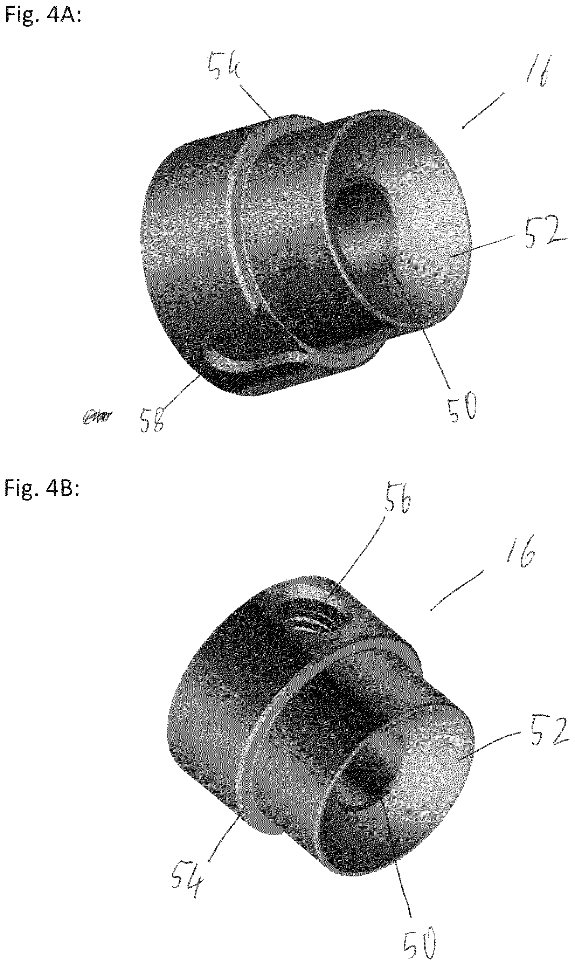

FIGs. 4A and 4B show a three-dimensional view of a gliding block 16. The gliding block 16 comprises an inward (i. e. concave) spherical section 52 and a hole 50 formed in the center of the inward spherical section 52. Upon assembly, a projection 44 is inserted into hole 50. While the present embodiment comprises two projections 44 that are inserted into holes 50 of the gliding blocks, respectively, the invention is not limited thereto, and the presence of only one projection to limit the range of motion may suffice. When being mounted to the joint member 12B, the inward spherical sections 52 of the gliding blocks form a concave spherical joint surface.

The gliding block 16 further comprises a step 55, which engages step 34 of mounting recess 30 upon insertion into the second component 10. Gliding block 16 further comprises a threaded hole 5, in which a screw 18 can be inserted to secure the gliding block 16 in the mounting recess 30 of the second component 10. Gliding block 16 also comprises a recess 58, which cooperates with projection 32 of the mounting recess 30 for preventing rotation of the gliding block 16 within the mounting recess 30 of the second component 10 and providing guidance during assembly. Insertion of the gliding block 16 is preferably facilitated by the opening of recess 58 being chamfered. Preventing rotation of the gliding block 16 also facilitates the insertion of screw 18 through hole 36 of the joint member of the second component 10 and into the threaded hole 56. More specifically, the illustrated configuration assists in aligning holes 36 and 56 upon insertion of gliding block 16 into mounting recess 30 due to the cooperation of recess 58 and projection 32.

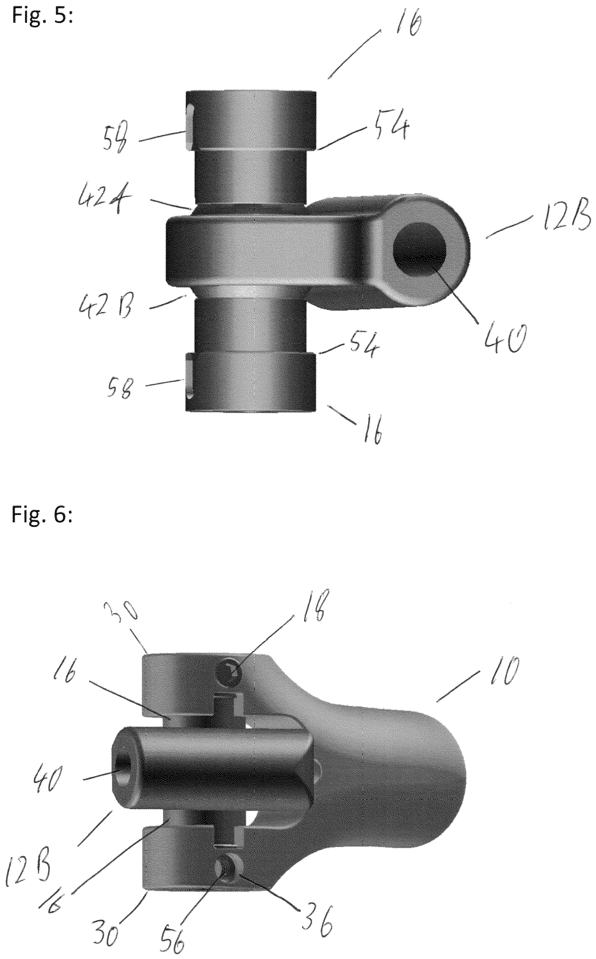

FIG. 5 is a perspective view showing the concave joint surface of the inward spherical sections 52 of the two gliding blocks 16 in contact with the convex joint surface of the outward spherical sections 42A and 42B of the joint member 12B. A similar configuration is shown in FIG. 7A, where the gliding blocks 16 are depicted in a transparent configuration for the detailed description of FIG. 7A further down below.

FIG. 6 is also a perspective view of two gliding blocks 16 being in engagement with the joint member 12B of the first component 12. In addition to FIG. 5, the second component 10 is shown and the gliding blocks 16 are located in the mounting recesses 30 of this component. One of the gliding blocks 16 is already secured via a screw 18 to the second component 10.

In the following, the varus-valgus movement of the first component 12 will be explained in more detail with reference to FIGs. 5, 7A and 7B. As described above, the outward spherical sections 42 of the first component 12 are configured to be in engagement (i. e. in contact) with the inward spherical sections 52 of the gliding blocks 16. Two projections 44 are inserted into two holes 50 of the gliding blocks 16, respectively.

FIG. 7B shows the varus-valgus movement of the first component 12 about the second pivotal axis, wherein the center configuration of FIG. 7B shows the first component 12 in a neutral position. Preferably, the projections 44 are substantially centered within holes 50 of the gliding blocks in said neutral position. The left and right configurations of FIG. 7B show the limits of the varus-valgus movement of the joint.

As explained above, the projections 44 serve to limit the varus-valgus movement of the first component 12. This limitation of the range of motion of the joint can be adjusted by adjusting the ratio of the diameter of the hole 50 of the gliding block 16 to the diameter of projections 44. The smaller the projections are in diameter in comparison to the diameter of hole 50, the more movement, i.e. range of motion in varus-valgus direction and rotation of the first component 12 is possible. In contrast, when the diameter of holes 50 of gliding blocks 16 and projections 44 are almost the same (i. e. they still allow a rotation about the first pivotal axis), hardly any movement is possible.

Limiting the range of motion via a projection 44 and a hole 50 is advantageous since it defines a limitation for the varus-valgus movement and distributes the abutment force more evenly. Further, allowing a defined amount of varus-valgus movement allows for a better adaptation to the kinematics of a patient's body, which in turn reduces wear of the joint, and at the same time provides predictable behavior of the joint (i. e. it prevents slackness of the joint).

It is beneficial to form the gliding blocks 16 out of UHMWPE and in particular X-Linked PE and/or Vitamin-E X-Linked PE. These materials have excellent properties for absorbing peak forces (e. g. when reaching the limits of the varus-valgus movement) and have advantageous wear characteristics. These materials have also proven to have the gliding performance needed for artificial joints.

It is particularly preferred to form the projections 44 with a conical shape so that the contact surface between the inner surface of hole 50 and the outer surface of projections 44 is basically established by a line contact.

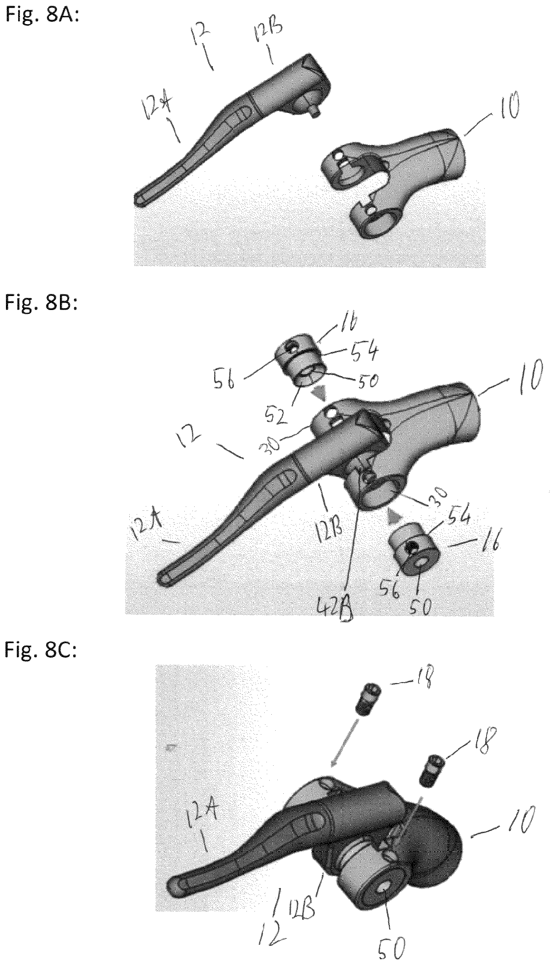

Now with reference to FIGs. 8A to 8C, the assembly of the joint prosthesis 1 will be explained. The first component 12 comprising the bone member 12A and the joint member 12B is inserted into the second component 10, i. e. into the yoke of the second component 10. In the illustrated embodiment, the prjections 44 of the joint member 12B comprising the outward spherical sections 42 are inserted through cut-out portions 38 for arranging the first component 12 between the two branches of the second component 10.

After insertion of the first component 12 into the second component 10, gliding blocks 16 are inserted into mounting recesses 30 of the second component 10 so as to engage with the outward spherical sections 42A and 42B as described above. Once the gliding blocks 16 are fully inserted into the mounting recesses 30, the gliding blocks are secured with screws 18 to the second component 10.

### REFERENCE SIGNS

- 1: Joint prosthesis
- 10: Second component
- 12: First component
- 12A: Bone member
- 12B: Joint member
- 14: Ball-and-socket-joint
- 16: Gliding block
- 18: Screw
- 30: Mounting recess
- 32: Projection hindering rotation
- 34: Step
- 36: Hole for screw
- 38: Cut out portion
- 40: Hole for bone member
- 42: Outward spherical section
- 44: Projection
- 50: Hole
- 52: Inward spherical section
- 54: Step
- 56: Threaded hole
- 58: Recess

## Claims

1. Joint prosthesis (1), the joint prosthesis (1) being configured to allow for flexural movement, comprising a first component (12) and a second component (10) being connected via a ball-and-socket-joint, wherein
the first component comprises two outward spherical sections (42), at least one of the outward spherical sections having a projection (44);
the ball-and-socket-joint comprises two gliding blocks (16) each having an inward spherical section (52) and at least one of the gliding blocks comprising a hole (50), the gliding blocks being attached to the second component,
the outward spherical sections are in gliding engagement with the inward spherical sections of the gliding blocks so that the first component is movably attached to the second component; and
the projection is inserted into the hole of one of the gliding blocks,
**characterized in that**
the joint prosthesis is further configured to allow for a defined varus-valgus movement,
wherein an inner diameter of the hole (50) is larger than an outer diameter of the projection (44) so as to define the varus-valgus movement.

2. Joint prosthesis (1) according to claim 1, wherein the projection (44) has a conical shape with the end of the conical projection away from the spherical section being smaller in circumference than the end of the conical projection at the spherical section.

3. Joint prosthesis (1) according to any one of the preceding claims, wherein the second one of the two outward spherical sections (42) has a second projection (44) and the second one of the two gliding blocks (16) also comprises a hole (50) into which hole the second projection is inserted, preferably the second projections is in a conical shape with the end of the conical projection away from the spherical section being smaller in circumference than the end of the conical projections at the spherical section.

4. Joint prosthesis (1) according to any one of the preceding claims, wherein the second component (10) comprises two mounting recesses (30) in which the gliding blocks (16) are inserted and attached.

5. Joint prosthesis (1) according to claim 4, wherein the gliding blocks (16) are secured with a screw in the mounting recesses (30).

6. Joint prosthesis (1) according to claim 4 or 5, wherein the mounting recesses (30) comprise a projection to hinder rotational movement of the gliding blocks (16).

7. Joint prosthesis (1) according to any of claims 4-6, wherein the mounting recess (30) comprises a means (34) to limit insertion of the gliding blocks (16) into the mounting recesses, the means preferably being a step or a pin.

8. Joint prosthesis (1) according to any one of the preceding claims, wherein the first component (12) comprises a bone member (12A) and a joint member (12B) comprising the outward spherical sections (42) and the at least one projection (44).

9. Joint prosthesis (1) according to any one of the preceding claims, wherein the two outward spherical sections (42) are arranged so that they form a spherical convex joint surface.

10. Joint prosthesis (1) according to any one of the preceding claims, wherein the second component (10) comprises one or two cut out portions (38) through which the at least one projection (44) of the first component (12) can be inserted at the time of assembly.

11. Joint prosthesis (1) according to any one of the preceding claims, wherein the diameter of curvature of the outward spherical sections (42) and the diameter of curvature of the inward spherical sections (52) are substantially equal.

12. Joint prosthesis (1) according to claim 8, wherein the joint member (12B), the outward spherical sections (42) and the at least one projection (44) are integrally formed as one member.

13. Joint prosthesis (1) according to any one of the preceding claims, wherein the joint prosthesis is an elbow joint prosthesis, in particular with the first component (12) being an ulnar component and the second component (10) being a humeral component.

14. Method of assembly of a joint prosthesis (1) according to any one of the preceding claims comprising the steps of introducing the first component (12) into the second component (10) and subsequently movably fixing the first component by inserting the gliding blocks (16).

15. Method of assembly of a joint prosthesis (1) according claim 14, wherein after insertion of the gliding blocks (16), the gliding blocks are secured to the second component (10) via a screw.

## Patentansprüche

1. Gelenkprothese (1), wobei die Gelenkprothese (1) dazu konfiguriert ist, eine Biegebewegung zu ermöglichen, umfassend eine erste Komponente (12) und eine zweite Komponente (10), die über ein Kugelgelenk verbunden sind, wobei
die erste Komponente zwei nach außen gerichtete Kugelabschnitte (42) umfasst, wobei mindestens einer der nach außen gerichteten Kugelabschnitte einen Vorsprung (44) aufweist;
das Kugelgelenk zwei Gleitblöcke (16) umfasst, die jeweils einen nach innen gerichteten Kugelabschnitt (52) aufweisen und mindestens einer der Gleitblöcke ein Loch (50) umfasst, wobei die Gleitblöcke an der zweiten Komponente angebracht sind,
die nach außen gerichteten Kugelabschnitte in Gleiteingriff mit den nach innen gerichteten Kugelabschnitten der Gleitblöcke stehen, sodass die erste Komponente beweglich an der zweiten Komponente angebracht ist; und
der Vorsprung in das Loch eines der Gleitblöcke eingesetzt ist, **dadurch gekennzeichnet, dass** die Gelenkprothese ferner dazu konfiguriert ist, eine definierte Varus-Valgus-Bewegung zu ermöglichen, wobei ein Innendurchmesser des Lochs (50) größer als ein Außendurchmesser des Vorsprungs (44) ist, um die Varus-Valgus-Bewegung zu definieren.

2. Gelenkprothese (1) nach Anspruch 1, wobei der Vorsprung (44) eine konische Form aufweist, wobei das vom Kugelabschnitt entfernte Ende des konischen Vorsprungs im Umfang kleiner als das Ende des konischen Vorsprungs an dem Kugelabschnitt ist.

3. Gelenkprothese (1) nach einem der vorhergehenden Ansprüche, wobei der zweite der beiden nach außen gerichteten Kugelabschnitte (42) einen zweiten Vorsprung (44) aufweist und der zweite der beiden Gleitblöcke (16) ebenfalls ein Loch (50) umfasst, in das der zweite Vorsprung eingesetzt wird, wobei die zweiten Vorsprünge vorzugsweise eine konische Form haben, wobei das vom sphärischen Abschnitt entfernte Ende des konischen Vorsprungs im Umfang kleiner als das Ende der konischen Vorsprünge an dem Kugelabschnitt ist.

4. Gelenkprothese (1) nach einem der vorhergehenden Ansprüche, wobei die zweite Komponente (10) zwei Einbauausnehmungen (30) aufweist, in die die Gleitblöcke (16) eingesetzt und an denen sie angebracht sind.

5. Gelenkprothese (1) nach Anspruch 4, wobei die Gleitblöcke (16) mit einer Schraube in den Einbauausnehmungen (30) befestigt sind.

6. Gelenkprothese (1) nach Anspruch 4 oder 5, wobei die Einbauausnehmungen (30) einen Vorsprung zur Behinderung einer Drehbewegung der Gleitblöcke (16) umfassen.

7. Gelenkprothese (1) nach einem der Ansprüche 4-6, wobei die Einbauausnehmung (30) ein Mittel (34) umfasst, um das Einsetzen der Gleitblöcke (16) in die Einbauausnehmungen zu begrenzen, wobei das Mittel vorzugsweise eine Stufe oder ein Stift ist.

8. Gelenkprothese (1) nach einem der vorhergehenden Ansprüche, wobei die erste Komponente (12) ein Knochenelement (12A) und ein Gelenkelement (12B) umfasst, das die nach außen gerichteten Kugelabschnitte (42) und den mindestens einen Vorsprung (44) umfasst.

9. Gelenkprothese (1) nach einem der vorhergehenden Ansprüche, wobei die beiden nach außen gerichteten Kugelabschnitte (42) so angeordnet sind, dass sie eine sphärische konvexe Gelenkfläche bilden.

10. Gelenkprothese (1) nach einem der vorhergehenden Ansprüche, wobei die zweite Komponente (10) einen oder zwei Ausschnitte (38) umfasst, durch die der mindestens eine Vorsprung (44) der ersten Komponente (12) zum Zeitpunkt der Montage eingesetzt werden kann.

11. Gelenkprothese (1) nach einem der vorhergehenden Ansprüche, wobei der Krümmungsdurchmesser der nach außen gerichteten Kugelabschnitte (42) und der Krümmungsdurchmesser der nach innen gerichteten Kugelabschnitte (52) im Wesentlichen gleich sind.

12. Gelenkprothese (1) nach Anspruch 8, wobei das Gelenkelement (12B), die nach außen gerichteten Kugelabschnitte (42) und der mindestens eine Vorsprung (44) einstückig als ein Element ausgebildet sind.

13. Gelenkprothese (1) nach einem der vorhergehenden Ansprüche, wobei die Gelenkprothese eine Ellenbogengelenkprothese ist, insbesondere wobei die erste Komponente (12) eine Ulnarkomponente und die zweite Komponente (10) eine Humeruskomponente ist.

14. Verfahren zur Montage einer Gelenkprothese (1) nach einem der vorhergehenden Ansprüche, umfassend die Schritte des Einbringens der ersten Komponente (12) in die zweite Komponente (10) und des anschließenden beweglichen Fixierens der ersten Komponente durch Einsetzen der Gleitblöcke (16).

15. Verfahren zur Montage einer Gelenkprothese (1) nach Anspruch 14, wobei nach dem Einsetzen der Gleitblöcke (16) die Gleitblöcke mittels einer Schraube an der zweiten Komponente (10) befestigt werden.

## Revendications

1. Prothèse d'articulation (1), la prothèse d'articulation (1) étant configurée pour permettre un mouvement de flexion, comprenant un premier composant (12) et un second composant (10) étant reliés par une rotule, dans laquelle
le premier composant comprend deux sections sphériques vers l'extérieur (42), au moins l'une des sections sphériques vers l'extérieur présentant une saillie (44) ;
la rotule comprend deux patins de glissement (16) présentant chacun une section sphérique vers l'intérieur (52) et au moins un des patins de glissement comprenant un trou (50), les patins de glissement étant fixés au second composant,
les sections sphériques vers l'extérieur sont en prise glissante avec les sections sphériques vers l'intérieur des patins de glissement de sorte que le premier composant est fixé de manière mobile au second composant ; et
la saillie est insérée dans le trou de l'un des patins de glissement, **caractérisée en ce que** la prothèse d'articulation est en outre configurée pour permettre un mouvement varus-valgus défini, dans laquelle un diamètre intérieur du trou (50) est plus grand qu'un diamètre extérieur de la saillie (44) de manière à définir le mouvement varus-valgus.

2. Prothèse d'articulation (1) selon la revendication 1, dans laquelle la saillie (44) présente une forme conique, l'extrémité de la saillie conique éloignée de la section sphérique étant plus petite en circonférence que l'extrémité de la saillie conique au niveau de la section sphérique.

3. Prothèse d'articulation (1) selon l'une quelconque des revendications précédentes, dans laquelle la seconde des deux sections sphériques extérieures (42) présente une seconde saillie (44) et le second des deux patins de glissement (16) comprend également un trou (50) dans lequel la seconde saillie est insérée, de préférence les secondes saillies présentent une forme conique, l'extrémité de la saillie conique éloignée de la section sphérique présentant une circonférence plus petite que l'extrémité des saillies coniques au niveau de la section sphérique.

4. Prothèse d'articulation (1) selon l'une quelconque des revendications précédentes, dans laquelle le second composant (10) comprend deux évidements de montage (30) dans lesquels les patins de glissement (16) sont insérés et fixés.

5. Prothèse d'articulation (1) selon la revendication 4, dans laquelle les patins de glissement (16) sont fixés de manière stable par une vis dans les évidements de montage (30).

6. Prothèse d'articulation (1) selon la revendication 4 ou 5, dans laquelle les évidements de montage (30) comprennent une saillie pour gêner le mouvement de rotation des patins de glissement (16).

7. Prothèse d'articulation (1) selon l'une quelconque des revendications 4 à 6, dans laquelle l'évidement de montage (30) comprend un moyen (34) pour limiter l'insertion des patins de glissement (16) dans les évidements de montage, le moyen étant de préférence une marche ou une épingle.

8. Prothèse d'articulation (1) selon l'une quelconque des revendications précédentes, dans laquelle le premier composant (12) comprend un élément osseux (12A) et un élément d'articulation (12B) comprenant les sections sphériques extérieures (42) et l'au moins une saillie (44).

9. Prothèse d'articulation (1) selon l'une quelconque des revendications précédentes, dans laquelle les deux sections sphériques extérieures (42) sont disposées de manière à former une surface d'articulation sphérique convexe.

10. Prothèse d'articulation (1) selon l'une quelconque des revendications précédentes, dans laquelle le second composant (10) comprend une ou deux parties découpées (38) à travers lesquelles l'au moins une saillie (44) du premier composant (12) peut être insérée au moment du montage.

11. Prothèse d'articulation (1) selon l'une quelconque des revendications précédentes, dans laquelle le diamètre de courbure des sections sphériques vers l'extérieur (42) et le diamètre de courbure des sections sphériques vers l'intérieur (52) sont sensiblement égaux.

12. Prothèse d'articulation (1) selon la revendication 8, dans laquelle l'élément d'articulation (12B), les sections sphériques vers l'extérieur (42) et l'au moins une saillie (44) sont formés d'un seul tenant en tant qu'un seul élément.

13. Prothèse d'articulation (1) selon l'une quelconque des revendications précédentes, dans laquelle la prothèse d'articulation est une prothèse d'articulation de coude, notamment avec le premier composant (12) étant un composant ulnaire et le deuxième composant (10) étant un composant huméral.

14. Procédé d'assemblage d'une prothèse d'articulation (1) selon l'une quelconque des revendications précédentes comprenant les étapes d'introduction du premier composant (12) dans le second composant (10) puis de fixation mobile du premier composant par insertion des patins de glissement (16).

15. Procédé d'assemblage d'une prothèse d'articulation (1) selon la revendication 14, dans lequel après insertion des patins de glissement (16), les patins de glissement sont fixés de manière stable au second composant (10) via une vis.
